# EUROPEAN PATENT APPLICATION

(11) **EP 1 201 202 A1**
(43) Date of publication of application: **02.05.2002**
(21) Application number: 00940915.2
(22) Date of filing: 03.07.2000
(51) Int. Cl.: A61F 2/04, A61L 27/00

(54) **ARTIFICIAL NEURAL TUBE**

(30) Priority: 07.07.1999 JP 19299399
(71) Applicant: Tapic International Co., Ltd., Tokyo 105-0001 (JP); Shimizu, Yasuhiko, Uji-shi, Kyoto 611-0002 (JP)
(72) Inventor: SHIMIZU, Yasuhiko, Uji-shi, Kyoto 611-0002 (JP)
(74) Representative: Stuart, Ian Alexander
(86) International application number: JP0004380
(87) International publication number: WO0103609

(57) **Abstract**

The present invention is to provide an artificial tube for nerve which remains in a body until a nerve regenerates, does not remain in the body as a foreign body after regeneration of the nerve, induces axons regenerated from severed nerve stumps, promotes infiltration of blood capillaries from the body and promotes regeneration of nerve tissue, and a method for producing the same. The artificial tube for nerve has fine fibrous collagen bodies (30) in the lumen of a tube comprised of a biodegradable and absorbable material, the voids inside the fine fibrous collagen bodies being filled with laminin.

## Description

### Technical Field

The present invention relates to an artificial tube for nerve.

### Background Art

In the case of peripheral nerve being severed surgically or severed due to injury, an initial attempt is made to directly anastomose the stumps of the severed peripheral nerve. In many cases, however, it is impossible to accurately anastomose the severed nerve directly resulting in the nerve being left in the severed state. Consequently, although the nerve attempts to regenerate towards the distal side, it is impaired by connective tissue. Hence, regeneration stops with the formation of a neuroma at the severed end without reaching the neural stump on the distal side. As a result, the function of the severed nerve is frequently not restored after the surgical wound or injury has healed, and sequella remain. In cases in which direct anastomosis is not possible, a peripheral nerve having a function which is not very important may be partially excised from the same patient, and autotransplantation may be performed to the severed site of the nerve using this peripheral nerve segment. However, in this method as well, not only are there many cases in which nerve function is not adequately restored, but there are also many cases in which decreased function is observed even at the portion at which the transplanted nerve is used.

Therefore, numerous attempts have been made to restore function by connecting the stumps of severed peripheral nerves with a tube-shaped material, namely an artificial tube for nerve, regenerating the axon from the stump on the central side of the nerve trunk towards the stump on the distal side, inducing the nerve to extend in the proper direction, and allowing the nerve to reach a myoneural junction or peripheral sensory receptor from the peripheral nerve trunk. In the past, various materials have been attempted to be used as artificial tube for nerve, examples of which include non-porous tubes made of silicone, polyethylene or polyvinyl chloride, porous tubes made of drawn polytetrafluoroethylene or cellulose, semi-permeable membrane tubes made of polyacrylonitrile or polysulfone, tubes made of biodegradable materials such as polyglycolic acid, polylactic acid or their copolymers, gelatin tubes, or biological tissue tubes originating in the same species such as arteries and veins. However, in regeneration experiments on peripheral nerves using these materials, since biological repair is impaired by the material, the length of nerve that has been able to be regenerated thus far has been at most on the order of 15 mm. In addition, not only is the regenerated nerve narrow without the form of the nerve being normally restored, but there are also many cases in which the function of regenerated nerve is not restored. In addition, although examples have been reported in which neural growth factor NGF is filled into a tube, since NGF ends up rapidly running out of the tube and dispersing, remarkable effects have not been obtained.

Although artificial tubes for nerve which comprise collagen tubes in which collagen fibers on which laminin and fibronectin are coated are filled (Tong, X., et al., Brain Research 663: 155-162 (1994), have recently been attempted, since the collagen tubes are unable to remain without being broken down until the nerve is regenerated to an adequate length, satisfactory results have not been obtained.

On the other hand, the spinal cord is considered to not regenerate once it has been damaged. In the case the spinal cord is damaged due to injury or tumor, the damaged spinal cord does not regenerate, and all function below the damaged portion is lost with paralysis remaining as the sequella. Recently however, experiments on animals have begun to be conducted that prove that the spinal cord is also able to regenerate. In the case the spinal cord is severed sharply and accurately re-sutured, function is restored and the damaged portion is repaired to a considerable degree. In addition, if a portion of the spinal cord is excised in the form of a tube and an intercostal nerve fasicle is implanted at that site, the portion of the spinal cord regenerates and function is at least partially restored. If a portion of the spinal cord is excised in the form of a tube, and fetal spinal cord is transplanted to that site, spinal cord function and form are restored. These findings have been observed in experiments in rats. In this case as well, it is recognized that regeneration occurs only in the case the transplanted fetal spinal cord segment is transplanted by properly aligning the respective neural processes. Based on the above findings, although it has been determined that regeneration of the spinal cord can occur by inducing the spinal cord so as to properly align the compartments of regenerated tissue, there have been no artificial tubes for spinal cord developed whatsoever that actually allow spinal cord regeneration.

Therefore, in order to control the rate of decomposition in the body so as to remain in the body until the nerve regenerates while also allowing degradation and absorption in the body as nerve regeneration progresses, the development of an artificial tube for nerve is desired that induces axons regenerated from severed nerve stumps to extend in the proper direction without pressing on the regenerated nerve following nerve regeneration, and causes rapid restoration of blood flow by promoting infiltration of blood capillaries from the body to promote regeneration of nerve tissue. In addition, there is also an urgent need for the development of an artificial tube for spinal cord that connects not only peripheral nerves but also the missing portions of spinal cord, and promotes proper regeneration of spinal cord tissue along with restoration of function.

### Disclosure of the Invention

The present invention relates to an artificial tube for nerve which is characterized in that the artificial tube for nerve has fine fibrous collagen bodies (30) in the lumen of a tube (10, 20) comprised of a biodegradable and absorbable material, the voids inside the fine fibrous collagen bodies being filled with laminin.

In addition, the present invention relates to a method for producing an artificial tube for nerve which is characterized in that the method comprises steps: preparing a tube (10, 20) comprised of a biodegradable and absorbable material, introducing a hydrochloric acid solution of collagen into the lumen of the tube, freezing and then freeze-drying the tube to form fine fibrous collagen bodies (30), performing thermal crosslinking treatment on the tube having the fine fibrous collagen bodies in its lumen, and introducing laminin into the fine fibrous collagen bodies.

### Brief Description of the Drawings

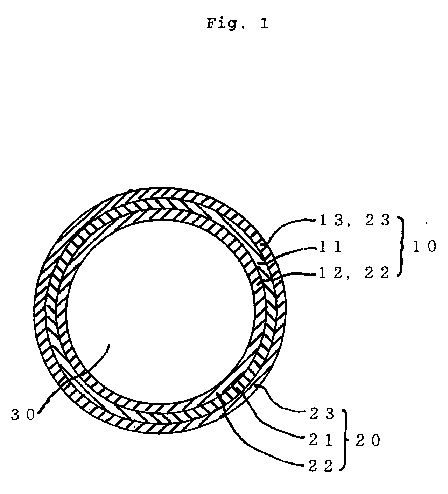

Fig. 1 is a drawing showing a cross-section of an embodiment of an artificial tube for nerve of the present invention (that provides a schematic representation of the structure without using actual dimensions. In addition, although the portion indicated by reference numeral 30 is an actual object, the diagonal lines are omitted for the sake of the explanation.)
Fig. 2 is a photograph (SEM photograph) showing the structure (cross-section) of an artificial tube for nerve of type 1 of the present invention.
Fig. 3 is a photograph (SEM photograph) showing the structure (cross-section) of tube base material of an artificial tube for nerve of type 2 of the present invention.

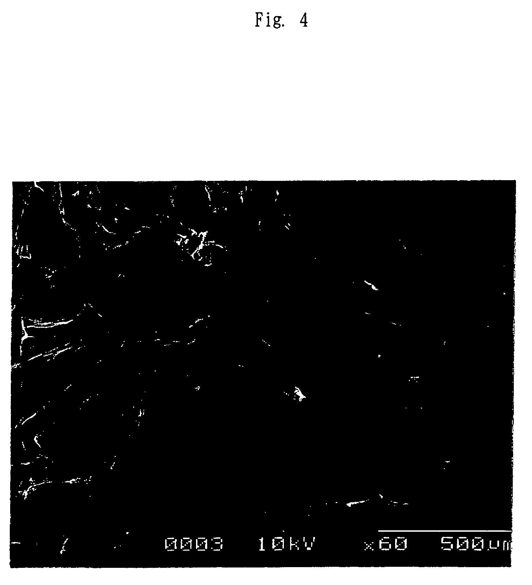

Fig. 4 is a photograph (SEM photograph) showing the structure (cross-section) of the fine fibrous collagen bodies present in the tube lumen of the artificial tube for nerve of the present invention.

The following reference numerals are used in the drawing.
11,21: (Biodegradable, absorbable material) tube
12,13: (Gelatin) coating layer
22,23: (Collagen) coating layer
30: Fine fibrous collagen bodies

### Best Mode for Carrying Out the Invention

Although the length and inner diameter of the tube (10, 20) that composes the artificial tube for nerve of the present invention differ according to the length and thickness of the severed portion of the nerve, in order to cover, for example, a missing portion on the order of about 25 mm of the sciatic nerve (using the example of a cat), the length is about 28-35 mm, and preferably about 30 mm, and the inner diameter is about 1-8 mm, and preferably about 4 mm. In addition, in the case of using the artificial tube for nerve of the present invention as an artificial tube for spinal cord as well, the length of the tube is determined according to the length of the severed portion, while the inner diameter is preferably about 2-12 mm and particularly preferably about 10 mm.

It is necessary that the tube (10, 20) composed of a material that is biodegradable and absorbable in vivo that composes the artificial tube for nerve of the present invention retains the shape of the tube to prevent invasion of body cells from outside the tube during the time until the severed nerve regenerates and the severed location is rejoined (about 1-3 months). Consequently, despite being biodegradable and absorbable in vivo, it is necessary that the material is able to retain its shape in the body for a certain period of time. Although examples of base materials of such a material include mesh materials selected from the group consisting of polyglycolic acid, polylactic acid, copolymer of glycolic acid and lactic acid, copolymer of lactic acid and ε-caprolactone, polydioxanone and copolymer of glycolic acid and trimethylene carbonate, a mesh tube comprised of polyglycolic acid is preferable. In addition, in addition to the above-mentioned mesh tube, a tube comprised of fine fibrous collagen can also be used preferably.

To begin with, a description is provided of the artificial tube for nerve of the present invention (hereinafter referred to as "Type 1") in which a tube comprised of a biodegradable and absorbable material has a coating layer (13, 23) comprised of gelatin or collagen on at least the outside of a mesh tube comprised of a material such as polyglycolic acid. In order to allow the mesh tube (11) comprised of a material such as polyglycolic acid to retain the shape of the tube for a period of about one to three months in the body, the thickness of the tube (referring to the thickness of the tube wall in the form of a cylinder, and to apply similarly hereinafter) is preferably about 0.1-3 mm, and particularly preferably about 0.5-2 mm. If the thickness exceeds about 3 mm, the tube obstructs regeneration of body tissue, and if the thickness is less than 0.1 mm, degradation and absorption of the tube proceed too rapidly, and the shape of the tube is not maintained until the nerve finishes regenerating. In addition, in the case of using the artificial tube for nerve of the present invention as an artificial tube for spinal cord, its thickness should preferably be about 0.2-5 mm, and particularly preferably about 0.5-3 mm.

In the case that the base material of the above tube is a material such as polyglycolic acid, said tube is in the form of a mesh to impart water permeability to the base material which is itself hydrophobic. The mesh pore size of this mesh tube (11) is preferably about 5-30 µm, and particularly preferably about 10-20 µm. If the mesh pore size is less than about 5 µm, cells and tissue are unable to proliferate, while if the mesh pore size exceeds about 30 µm, entry of tissue becomes excessive.

In addition, since said material itself does not have an action that promotes tissue regeneration, although it is made to have a coating layer (13, 23) comprised of a material having action that promotes tissue regeneration on at least the outside of tube (11) serving as the base material, it is preferably coated or filled with a material having action that promotes tissue regeneration on both the inside and outside of the tube serving as said base material and inside the mesh pores. The thickness of the coating layers (13,23 and/or 12,22) is preferably about 0.2-5 mm, and particularly preferably 0.5-3 mm. Examples of such materials that promote tissue regeneration include collagen or gelatin which have water-permeability, do not cause foreign body reactions when applied in the body, have excellent bioaffinity and tissue compatibility, and have an action that promotes tissue regeneration. Collagen originating in various animals conventionally used in the past can be used for the collagen raw material, preferable examples of which include type I collagen or a mixture of type I and type III collagen originating in the skin, bone, cartilage, tendon and organs of cows, pigs, rabbits, sheep, kangaroos or birds that is solubilized by acid, base, enzymes and so forth. The coating layers composed of collagen are layers having an amorphous structure in which collagen molecules are dispersed. Purified gelatin according to the Japanese Pharmacopoeia can be used for the raw material of a coating layer composed of gelatin.

In the artificial tube for nerve of the present invention, the tube base material composed of a material that is biodegradable and absorbable in vivo can be the mesh tube (11) composed of a material such as the above-mentioned polyglycolic acid, or a tube (21) composed of fine fibrous collagen having collagen having an action of promoting tissue regeneration for its raw material. The following provides a description of the artificial tube for nerve of the present invention (hereinafter referred to as "Type 2") in which the material that is biodegradable and absorbable in vivo is a tube composed of fine fibrous collagen, and the coating layer (23 and/or 22) possessed on at least the outside of said tube is composed of collagen.

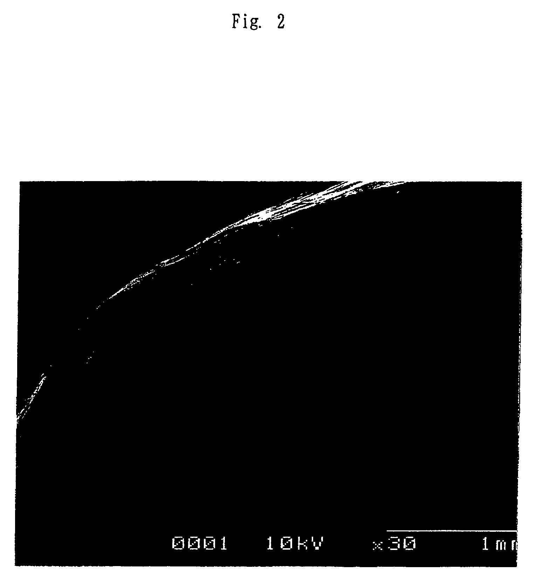

Type I collagen or a mixed collagen of type I and type III of animal origin like that has been used in the past and is solubilized by acid, base or enzymes and so forth in the same manner as the raw material of the coating layer of the artificial tube for nerve of type 1 is preferable for the collagen used for the raw material of said tube base material. This material composed of fine fibrous collagen is a matrix or thread-like woven or knitted product of a non-woven fabric-like multi-element structure in which fine fibers composed of collagen molecules are overlapped in multiple layers (and more specifically, using microfibers having a diameter of 3-7 nm composed of several collagen molecules as the basic unit, said microfibers are bundled to form ultrafine fibers having a diameter of 30-70 nm, said ultrafine fibers are further bundled to form fine fibers having a diameter of 1-3 µm, rows of bundles of said fine fibers are laminated vertically and horizontally in alternating fashion to form fibers having a diameter of 5-8 µm, and said fibers are fallen on top of one another in a coaxial direction to form sheet fibers having a diameter of 20-50 µm, ultimately resulting in the formation a fibrous collagen as the maximum unit by randomly intermingling these sheet fibers 11; see Fig. 2). Tube (21) that uses this for its material has an inner diameter and length similar to tube (11) of the artificial tube for nerve of type 1. Its thickness is preferably about 0.5-5 mm, and particularly preferably 1-2 mm. In addition, in the case of using the artificial tube for nerve of the present invention as an artificial tube for spinal cord, its thickness is preferably about 0.5-5 mm, and particularly preferably about 1-3 mm. In addition, the coating layer (23 and/or 22) composed of collagen formed on at least the outside of this tube (21) uses conventional solubilized type I or a mixed collagen of type I and type III of animal origin for its raw material similar to the non-woven fabric-like multi-element structure composed of fine fibrous collagen for the tube base material. However, the form is that of a layer having an amorphous structure in which collagen molecules are dispersed. Furthermore, the thickness of the coating layer is preferably about 0.1-2 mm, and particularly preferably about 0.5-1 mm.

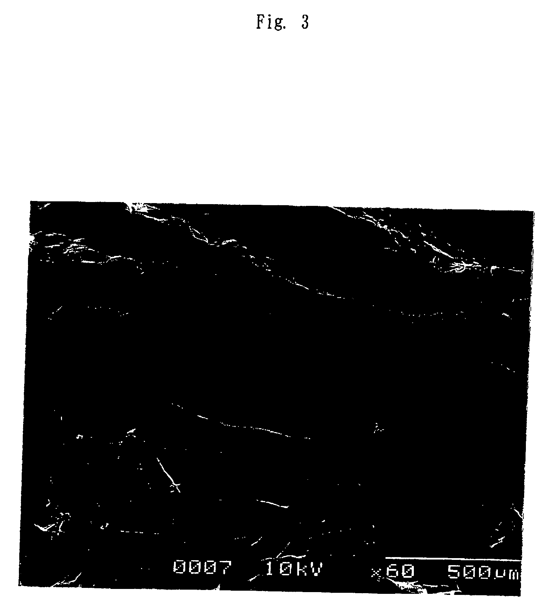

As previously described in details, the artificial tube for nerve of the present invention has fine fibrous collagen bodies (30) in the lumen of a tube (10,20) composed of a biodegradable and absorbable material, and laminin is filled in the voids in said fine fibrous collagen bodies (here, said fine fibrous collagen bodies have a structure that is substantially similar to the non-woven fabric-like multi-element structure composed of fine fibrous collagen serving as the tube base material; see Fig. 3). When this artificial tube for nerve is applied in the body, nerve fibers use said fine fibrous collagen bodies as footholds for regeneration for the purpose of regenerating and extending. (Furthermore, said fine fibrous collagen bodies are gradually digested and destroyed during the course of regeneration and extension of nerve fibers.)

As a preferable mode of the present invention, the tube base material (11 or 21) composed of a material that is biodegradable and absorbable in vivo is a tube (11) composed of a cylindrical mesh body made of polyglycolic acid, and the coating layer (23 and/or 22) of said tube is composed of amorphous collagen.

The following provides a description of the method for producing the artificial tube for nerve of the present invention. To begin with, in order to produce the artificial tube for nerve of type 1, a mesh tube (11) is first produced using a material such as polyglycolic acid. Although this may be produced by any method, as an example of such a method, fibers of polyglycolic acid and so forth (fibers having a diameter of, for example, 0.1 mm) are woven into the shape of a cylinder to obtain a mesh tube having the above thickness. The prepared mesh tube (11) is then coated with the above collagen or gelatin solution or immersed in said solution (this coating or immersion is to be hereinafter referred to as "Coating") and then air-dried to form a collagen or gelatin coating layer (13,23 and/or 12,22) on at least the outside of mesh tube (11) and inside the mesh pores (in the case of forming said collagen or gelatin coating layer only on the outside of said mesh tube and inside the mesh pores, a rod made of Teflon and so forth that makes contact with the inside of said mesh tube should be inserted into the lumen of said mesh tube prior to coating of said collagen or gelatin solution). In order to form this collagen or gelatin coating layer, an approximately 1 N hydrochloric acid solution (pH of about 3) preferably containing about 1-3 wt%, and particularly preferably about 1-2 wt%, of collagen, or preferably an about 2-30 wt%, and particularly preferably about 10-20 wt%, aqueous gelatin solution is used. Furthermore, in this type of artificial tube for nerve, it is convenient to coat mesh tube (11) with collagen or gelatin after treating with plasma discharge, ozone emission or other hydrophilization treatment to impart mesh tube (11) with hydrophilic properties.

On the other hand, in order to prepare the artificial tube for nerve of type 2, a rod made of Teflon and so forth that makes contact with the inside of the tube and has, for example, a diameter of about 1-8 mm, and preferably about 4 mm, is used for the core. Furthermore, in the case of using the artificial tube for nerve of the present invention as an artificial tube for spinal cord, the rod having a diameter of preferably about 2-12 mm, and particularly preferably about 10 mm, is used. The core is immersed in an approximately 1 N hydrochloric acid solution containing preferably about 0.5-3 wt%, and particularly preferably about 1-2 wt%, of collagen, and a collagen hydrochloric acid solution layer having a thickness of preferably about 5-20 mm, and particularly preferably about 10 mm, is formed on the surface of said core followed by freezing (for example, at about 0°C for about 12 hours). Furthermore, in the case of using the artificial tube for nerve of the present invention as an artificial tube for spinal cord, a collagen hydrochloric acid solution layer is formed having a thickness of preferably about 5-30 mm, and particularly preferably about 20 mm, followed by freezing. As a result of freezing, fine fragments of ice form between the collagen molecules dispersed in the hydrochloric acid solution, phase separation occurs in the collagen hydrochloric acid solution, and fine fibers of collagen are formed due to rearrangement of the collagen molecules. Next, this is further freeze-dried (for example, at about 0°C for about 24 hours) in a vacuum. As a result of freeze-drying, in addition to the fine ice fragments between the collagen molecules vaporizing, a tube is obtained composed of a non-woven fabric-like collagen layer in which fine fibers of collagen overlap in multiple layers.

Next, the core on which is formed this fine fibrous collagen layer is placed in a pouch made of polyethylene and so forth, sealed and degassed or not degassed followed by mechanical pressing of said fine fibrous collagen layer to compress the collagen layer. As a result of compressing, high-density, fine fibrous collagen layer (21) is obtained. This compression procedure is performed such that the thickness of said fine fibrous collagen layer after compression is preferably about 0.5-5 mm, and particularly preferably about 1-2 mm, or in the case of using as an artificial tube for spinal cord, the compression procedure is performed such that the thickness of the fine fibrous collagen layer after compression is preferably about 0.5-5 mm, and particularly preferably about 1-3 mm. Furthermore, in the case of using that in which a collagen thread-like product is woven or knitted for the tube composed of fine fibrous collagen, in place of forming the above collagen hydrochloric acid solution layer, wet spinning is performed to first produce a collagen thread-like product from the above collagen hydrochloric acid solution after which this is woven or knitted into the shape of a tube. The remainder of the procedure starting with freezing is the same as that described above.

Collagen coating layer (23 and/or 22) is further formed on at least the outside of compressed fine fibrous collagen layer (21) formed in this manner. As a result of forming these collagen coating layers (23 and/or 22), a tube (20) composed of a biodegradable and absorbable material is obtained having even greater strength. In order to form these collagen coating layers (23 and/or 22), the tube composed of fine fibrous collagen layer (21) removed from the above-mentioned rod or core is preferably again coated with or immersed in an approximately 1 N hydrochloric acid solution containing preferably about 0.5-3 wt%, and particularly preferably about 1-2 wt%, collagen, and a collagen hydrochloric acid solution layer is formed on at least the outside of fine fibrous collagen layer (21) followed by air-drying. This coating or immersion and air-drying procedure is repeated several times, and preferably 5-20 times, to obtain a collagen coating layer (23 and/or 22) having an amorphous structure in which collagen molecules are dispersed (the thickness of the collagen hydrochloric acid solution layer is preferably about 0.2-1.0 mm, and particularly preferably about 0.5 mm, overall). In the case of using the artificial tube for nerve of the present invention as an artificial tube for spinal cord, the thickness is the same.

Tube (20) prepared in this manner can be handled easily and allows easy suturing with nerves due to its high tear strength as compared with a tube consisting of compressed fine fibrous collagen layer (21) alone (due to partial entry of amorphous collagen into said compressed fine fibrous collagen layer and partial dissolution and precipitation of collagen at the interface of said compressed fine fibrous collagen layer and said collagen coating layer).

Fine fibrous collagen bodies (30) are formed in the lumen of tube (10,20) composed of a biodegradable and absorbable material prepared as described above. Formation of these fine fibrous collagen bodies (30) should be performed in the same manner as formation of the tube (21) of type 2 with the exception of not performing the core filling and compression procedure. In other words, the above collagen hydrochloric acid solution is poured into the lumen of these tubes using tube (10) or tube (20) as a kind of mold followed by freezing and freeze-drying.

Furthermore, prior to formation of these fine fibrous collagen bodies (30), crosslinking treatment is performed in order to impart resistance to water-solubility to tube (21) composed of the collagen or gelatin coating layer (13,23 and/or 12,22) and compressed fine fibrous collagen (in the case of type 2, this crosslinking treatment may also be performed after preparing tube (21) and before formation of the coating layer (23 and/or 22).
Crosslinking treatment is advantageous for the artificial tube for nerve of the present invention because it maintains the shape of the tube and prevents invasion of cells from outside the artificial tube for nerve during the time until the peripheral nerve is finished regenerating.

Although varying according to the length of the severed nerve portion that requires regeneration (since the imparting of the shape retention function of the tube serves as the rate-determining step in the body), crosslinking treatment is performed to an extent that the shape of the tube is retained for 1-3 months after application in the body. Although examples of crosslinking methods include gamma ray crosslinking, ultraviolet ray crosslinking, electron beam crosslinking, thermal dehydration crosslinking, glutaraldehyde crosslinking, epoxy crosslinking and water-soluble carbodiimide crosslinking, thermal dehydration crosslinking is preferable because it is easy to control the degree of crosslinking and does not have an effect on the body even when used for crosslinking treatment. The crosslinking treatment is performed in a vacuum at a temperature of, for example, about 105-150°C, preferably about 120-150°C, and particularly preferably about 140°C, for, for example, about 6-24 hours, preferably about 6-12 hours, and particularly preferably about 12 hours.

Finally, a component that aids the growth of nerve fibers is filled into the voids in the above fine fibrous collagen bodies (30). Preferable examples of such a component include laminin, and particularly preferably human laminin. As an example of a filling method, tube (10,20) having fine fibrous collagen bodies (30) in its lumen is immersed in a solution of laminin dissolved in PBS (Phosphate Buffered Saline), or a PBS solution of laminin is injected into said fine fibrous collagen bodies. However, prior to this laminin filling step, crosslinking treatment, and preferably thermal dehydration crosslinking treatment, is preferably performed on the said fine fibrous collagen bodies produced for the same reasons as in the production step of fine fibrous collagen bodies (30). Furthermore, in the case of using the artificial tube for nerve of the present invention as an artificial tube for spinal cord, an additional component for promoting regeneration and extension of nerve fibers, such as at least one among cell nutrient/growth factors like TGF-β, inflammatory cells including autologous macrophages (cultured in vitro) and autologous, homologous or heterologous myelin (medullary sheath) forming cells such as oligodendroglia and Schwann cells, are preferably introduced into said fine fibrous collagen bodies in addition to this laminin filling. Introduction of these additional components should be carried out in accordance with routine methods. After filling and introducing these components that aid in nerve regeneration and extension, the entire structure is air-dried to complete production of the artificial tube for nerve of the present invention (naturally, this does not mean that procedures required for distribution on the market, such as packaging and sterilization, do not have to be carried out).

The artificial tube for nerve prepared in the manner described above can be used to restore nerve function by inserting both stumps of a nerve that has been severed by injury or surgical procedure into the present artificial tube for nerve and fixing those portions by knotting suture to induce axon regeneration and extension in the proper direction, and allow axons to reach from the peripheral nerve trunk to a neuromuscular junction or peripheral sensory receptor. In addition, in the case the spinal cord is damaged due to injury as well, by removing the vertebrae corresponding to the damaged portion and covering the damaged portion of the spinal cord with the present artificial tube for nerve, it is believed that the damaged spinal cord can be regenerated and its function restored.

Although the following provides a detailed explanation of the present invention through its examples and comparative examples, the present invention is not limited to these.

### Example

Polyglycolic acid (PGA) fibers (diameter: 0.1 mm) were woven into a tubular shape to prepare a polyglycolic acid mesh tube (mesh pore size: approximately 10-20 µm) having a length of about 100 mm, inner diameter of about 4-5 mm and thickness of about 1 mm. By making its surface hydrophilic by subjecting to plasma discharge treatment and immersing this mesh tube in 1 N hydrochloric acid solution containing 1.0 wt% enzyme-solubilized collagen originating in pig skin and then air-drying, the inside and outside of the tube were coated with said collagen hydrochloric acid solution (and naturally the insides of the mesh pores were also filled with said collagen hydrochloric acid solution; here, the immersion and drying procedure was repeated 10 times).

Next, after performing thermal dehydration crosslinking treatment (140°C x 24 hr) on the above tube having collagen coating layers on its inside and outside, the above collagen hydrochloric acid solution was poured into its lumen followed by freezing (-20°C x 24 hr), freeze-drying (-80°C x 48 hr under vacuum) and performing thermal dehydration crosslinking treatment (140°C x 24 hr) again.

The above tube having fine fibrous collagen bodies in its lumen following crosslinking treatment obtained in this manner was immersed in a PBS solution of human laminin (concentration: 10 µg/ml) followed by air-drying (this procedure was repeated three times) to obtain the artificial tube for nerve of the present invention (Type 1).

80 mm of the common peroneal nerve of dogs (body weight: 10 kg) was excised, the nerve stumps on both ends were inserted into the above-mentioned artificial tube for nerve and the overlapping portions of said artificial tube for nerve and said nerve stumps were fixed by knotting suture with 10-0 Nylon thread followed by evaluation over time.

### Comparative Example

Enzyme-solubilized collagen fibers originating in pig skin (diameter: about 5 µm) subjected in advance to thermal dehydration crosslinking treatment (140°C x 24 hr) were immersed in a PBS solution of human laminin (concentration: 10 µg/ml) followed by air-drying (this procedure was repeated three times) to obtain about 80 laminin-coated collagen fibers that were inserted into the lumen of the above PGA mesh tube having collagen coating layers on its inside and outside so that the fibers were substantially parallel to the axis of said tube, and observations similar to the example were conducted using the resulting artificial tube for nerve.

### Observation Results

In the case of the comparative example, abnormal stance of the affected rear paw when stationary and claudication when walking were observed at one month after surgery, and delay of the above stance and walking abnormality were observed in the majority of the animals even at three months after surgery. In contrast, in the example, similar functional abnormalities were observed to be diminished at one month after surgery, and both had nearly completely disappeared at three months after surgery. According to the results of electrophysiological tests, the amount of time from disappearance of response immediately after surgery to re-induction became shorter for both the somatosensory electric potential (SEP), which expresses recovery of sensory nerves, and the compound muscle activation potential (CMAP), which expresses recovery of motor nerves, and the recovery index was also promoted at three months after surgery.

### Industrial Applicability

The artificial tube for nerve of the present invention is able to retain its shape until the nerve finishes regenerating. In addition, since it induces and promotes nerve regeneration, severed nerves regenerate faster and longer in comparison with conventional artificial tubes for nerve, the state of the regenerated nerve more closely approaches the normal state, and recovery of nerve function is also favorable. In addition, it can also be used as an artificial tube for spinal cord for regeneration and recovery of damaged spinal cord.

## Claims

1. An artificial tube for nerve having fine fibrous collagen bodies in the lumen of a tube comprised of a biodegradable and absorbable material, the voids inside the fine fibrous collagen bodies being filled with laminin.

2. The artificial tube for nerve according to claim 1, wherein the biodegradable and absorbable material is a mesh material composed of a material selected from the group consisting of polyglycolic acid, polylactic acid, copolymer of glycolic acid and lactic acid, copolymer of lactic acid and ε-caprolactone, polydioxanone and copolymer of glycolic acid and trimethylene carbonate, and has a coating layer composed of gelatin or collagen on at least the outside of said tube.

3. The artificial tube for nerve according to claim 2, wherein the mesh material has a mesh pore size of about 5-30 µm.

4. The artificial tube for nerve according to claim 1, wherein the biodegradable and absorbable material is composed of fine fibrous collagen, and has a coating layer composed of collagen at least on the outside of said tube.

5. The artificial tube for nerve according to any of claims 1 to 4, wherein at least one of cell nutrient/growth factors, autologous inflammatory cells or autologous, homologous or heterologous myelin forming cells are additionally introduced into the fine fibrous collagen bodies.

6. A method for producing an artificial tube for nerve comprising steps: preparing a tube comprised of a biodegradable and absorbable material, introducing a hydrochloric acid solution of collagen into the lumen of the tube, freezing and then freeze-drying the tube to form fine fibrous collagen bodies, performing thermal crosslinking treatment on the tube having the fine fibrous collagen bodies in its lumen, and introducing laminin into the fine fibrous collagen bodies.

7. The method according to claim 6, wherein the tube comprised of a biodegradable and absorbable material is obtained by coating a gelatin or collagen solution onto at least the outside of a mesh material composed of a material selected from the group consisting of polyglycolic acid, polylactic acid, copolymer of glycolic acid and lactic acid, copolymer of lactic acid and ε-caprolactone, polydioxanone and copolymer of glycolic acid and trimethylene carbonate, air-drying and subjecting to thermal crosslinking treatment.

8. The method according to claim 6, wherein the tube comprised of a biodegradable and absorbable material is obtained by coating a hydrochloric acid solution of collagen onto the surface of a core material, freezing and then freeze-drying to obtain a layer composed of fine fibrous collagen, compressing the fine fibrous collagen layer, coating a gelatin or collagen solution onto at least the outside of the compressed fine fibrous collagen layer, air-drying and subjecting to thermal crosslinking treatment.

9. The method according to either of claims 7 or 8, wherein at least one kind of cell nutrient/growth factors, separately cultured autologous inflammatory cells or autologous, homologous or heterologous myelin forming cells loaded onto gelatin or collagen are introduced into the fine fibrous collagen bodies into which laminin has been introduced.
